(19) Europäisches Patentamt
European Patent Office
Office européen des brevets

(11) Publication number: **0 509 442 A1**

# (12) EUROPEAN PATENT APPLICATION

(21) Application number: **92106420.0**

(51) Int. Cl.5: **A61K 37/64**, C07K 15/00

(22) Date of filing: **14.04.92**

(30) Priority: **16.04.91 US 685939**

(43) Date of publication of application:
**21.10.92 Bulletin 92/43**

(84) Designated Contracting States:
**PT**

(71) Applicant: **SCHERING CORPORATION**
**2000 Galloping Hill Road**
**Kenilworth New Jersey 07033(US)**

(72) Inventor: **Neustadt, Bernard R.**
**24 Brook Place**
**West Orange, New Jersey 07052(US)**

(74) Representative: **von Kreisler, Alek, Dipl.-Chem. et al**
**Patentanwälte Von Kreisler-Selting-Werner,**
**Deichmannhaus am Hauptbahnhof**
**W-5000 Köln 1(DE)**

## (54) Use of neutral endopeptidase inhibitors in the treatment of nephrotoxicity.

(57) Treatment and prevention of immunosuppression therapy-induced nephrotoxicity with neutral endopeptidases such as N-[N-[(L)-[1-[(2,2-dimethyl-1,3-dioxolan-4-yl)-methoxy]carbonyl]-2-phenylethyl]-L-phenylalanyl]-β-alanine and N-[2-mercaptomethyl-3-(2-methylphenyl)propioyl]-methionine are disclosed.

EP 0 509 442 A1

## BACKGROUND OF THE INVENTION

The present invention relates to the treatment and prevention of nephrotoxicity induced by immunosuppression therapy by administration of a neutral endopeptidase (NEP) inhibitor.

The invention also relates to a pharmaceutical composition comprising an NEP inhibitor for use in treating or preventing immunosuppression induced nephrotoxicity.

Cyclosporin A is a fungally-derived peptide immunosuppressant used in the treatment of allogenic graft and transplant rejections and in the treatment of immune-related diseases. An unfortunate side effect of cyclosporin therapy is nephrotoxicity, evidenced by decreases in glomerular filtration rate, urinary output and sodium excretion.

FK-506, a macrocyclic immunomodulator useful in the treatment of allogenic graft and transplant rejection, is reported in Annual Reports in Medicinal Chemistry, vol. 25 (1989), p. 195-204.

Atrial natriuretic peptides (ANP) are a family of vasodilator, diuretic and antihypertensive peptides which have been the subject of many recent reports in the literature, for example Annu. Rev. Pharm. Tox.. 29. - (1989) p. 23-54. The function of ANP is to maintain salt and water homeostasis as well as to regulate blood pressure. ANP is rapidly deactivated in the circulation by at least two processes: a receptor-mediated clearance as reported in Am. J. Physiol.. 256 ((1989) p. R469-R475 and an enzymatic inactivation via NEP reported in Biochem. J.. 243 (1987) p. 183-187. Capasso et al reported in Am. J. Hyper.. 3, 3 (1990) p. 204-210 that administration of ANP to rats exposed to cyclosporin treatment reversed the nephrotoxic effects.

NEP (EC 3.4.24.11; enkephalinase; atriopeptidase) is a zinc-containing metalloprotease which cleaves a variety of peptide substrates on the amino terminal side of aromatic amino acids. See Biochem. J.. 241 - (1987) p. 237-247. Substrates for this enzyme include, but are not limited to, ANP, brain natriuretic peptide, met and leu enkephalin, bradykinin, neurokinin A, and substance P. It has been previously demonstrated that inhibitors of NEP potentiate the hypotensive, diuretic, natriuretic and plasma ANP responses to pharmacological injection of ANP in experimental animals. The potentiation of ANP by two specific NEP inhibitors is reported by Sybertz et al in J. Pharmacol. Exp. Ther.. 250, 2 (1989) p. 624-631, and in Hypertension. 15, 2 (1990) p. 152-161, while the potentiation of ANP by NEP inhibitors in general was disclosed in U.S. patent 4,749,688. In U.S. 4,740,499, Olins disclosed the use of thiorphan and kelatorphan to potentiate atrial peptides.

## SUMMARY OF THE INVENTION

The present invention relates to a method of treating or preventing nephrotoxicity induced by immunosuppression therapy comprising administering an effective amount of an NEP inhibitor to a mammal in need of such treatment. In particular, the invention relates to treatment or prevention of nephrotoxicity induced by administration of an immunosuppressant cyclosporin or FK-506.

Another aspect of the invention relates to pharmaceutical compositions comprising an amount of an NEP inhibitor effective to treat or prevent nephrotoxicity in a pharmaceutically acceptable carrier.

A third aspect of the invention relates to kits comprising an immunosuppressant and an NEP inhibitor in separate compositions.

## DETAILED DESCRIPTION

The NEP inhibitors suitable for use in this invention include carboxyalkyl dipeptides disclosed in U.S. patent 4,610,816, having the formula

$R_1{}^aCH(COR_2{}^a)$-NH-$CHR_3{}^a$-$CONH(CH_2)_p{}^a$-$C(R_4{}^aR_5{}^a)$-$COR_6{}^a$

wherein:

$R_1{}^a$ is alkyl having from 1 to 6 carbon atoms, adamantylmethyl, cycloalkylmethyl having from 4 to 8 carbon atoms or $A^a$-$X^a{}_m{}^a$-$C_n{}^aH_{2n}{}^a$- {wherein $X^a$ is oxygen or sulfur; $A^a$ is phenyl which may be substituted with the group $Y^a$ (wherein $Y^a$ is halogen, hydroxy, trifluoromethyl, alkoxy having from 1 to 6 carbon atoms, 2- or 3-furanyl, 2- or 3-thienyl, or phenyl (which may be substituted with halogen, hydroxy, trifluoromethyl, alkoxy having from 1 to 6 carbon atoms or alkyl having from 1 to 6 carbon atoms)), benzyl (the phenyl ring of which may be substituted with the group $Y^a$ as defined herein), 1- or 2-naphthyl, 2- or 3-furanyl or 2- or 3-thienyl; $m^a$ is 0 or 1; and $n^a$ is 0, 1, 2, 3 or 4};

$R_2{}^a$ and $R_6{}^a$ may be the same or different and each is independently selected from hydroxy, alkoxy

having from 1 to 8 carbon atoms, $B^a$-$X^a{}_m{}^a$-$C_n{}^aH_{2n}{}^a$-O- {wherein $B^a$ is phenyl (which may be substituted with the group $Y^a$ as defined herein) or 1- and 2-naphthyl; and $X^a$, $m^a$ and $n^a$ are as defined herein provided that, when $n^a = 0$, $m^a$ is zero}, -$OCH_2OCO$-alkyl having from 3 to 8 carbon atoms, -$OCH_2CO$-phenyl {the phenyl ring of which may be substituted with the group $Y^a$ as defined herein}, 1-glyceryl,

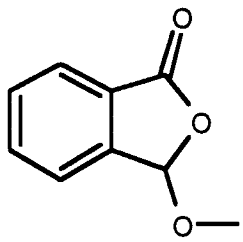

or

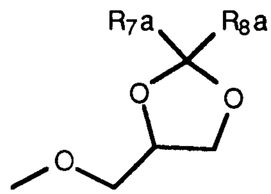

{wherein $R_7{}^a$ is hydrogen, alkyl having from 1 to 6 carbon atoms, or phenyl which may be sustituted with the group $Y^a$ as defined herein, and $R_8{}^a$ is hydrogen or alkyl having from 1 to 6 carbon atoms};

$R_2{}^a$ may also be -$NR_7{}^aR_8{}^a$ wherein $R_7{}^a$ and $R_8{}^a$ are as defined herein;

$R_3{}^a$ is alkyl having from 1 to 6 carbon atoms, cycloalkylmethyl having from 4 to 8 carbon atoms, 2- or 3-thienylmethyl, 2- or 3-furanylmethyl, 1- or 2-naphthylmethyl, or benzyl the phenyl ring of which may be substituted with the group $Y^a$ as defined herein;

$R_4{}^a$ is $D^a$-$C_n{}^aH_{2n}{}^a$-$O_m{}^a$- wherein $D^a$ is selected from hydrogen, alkyl having from 1 to 4 carbon atoms or phenyl which may be substituted with the group $Z^a$; {wherein $Z^a$ is halogen, hydroxy, trifluoromethyl, alkoxy having from 1 to 6 carbon atoms, or alkyl having from 1 to 6 carbon atoms}; and $m^a$ and $n^a$ are as defined herein;

$R_4{}^a$ may also be -$NR_5{}^aCOR_7{}^a$ {wherein $R_5{}^a$ and $R_7{}^a$ are as defined herein}, or -$NR_5{}^aCO_2R_9{}^a$ {wherein $R_5{}^a$ is as defined herein and $R_9{}^a$ is alkyl having from 1 to 6 carbon atoms or phenyl which may be substituted with the group $Y^a$ as defined herein};

$R_5{}^a$ is hydrogen or alkyl having from 1 to 6 carbon atoms; and p is 1 or 2;

or pharmaceutically acceptable addition salts thereof; wherein preferred compounds are N-[N-[(L)-[1-[-(2,2-dimethyl-1,3-dioxolan-4-yl)-methoxy]carbonyl]-2-phenylethyl]-L-phenylalanyl]-β-alanine and N-[N-[(L)-1-carboxy-2-phenylethyl]-L-phenylalanyl]-β-alanine;

mercaptoacyl amino acids disclosed in U.S. patent 4,801,609, having the formulae

$Q^bS$-$CH_2$-CH(-$(CH_2)_n{}^b$-$R^{1b}$)-C(O)-NH-CH($R^{2b}$)-C(O)-$R^{3b}$ Ib

$Q^bS$-$CH_2$-CH(-$(CH_2)_n{}^b$-$R^{1ab}$)-C(O)-NH-CH($R^{2ab}$)-C(O)-$R^{3b}$ IIb

$Q^bS$-$CH_2$-CH(-$(CH_2)_n{}^b$-$R^{1ab}$)-C(O)-NH-CH($R^{2b}$)-C(O)-$R^{3ab}$ IIIb

wherein:

$R^{1b}$ is phenyl substituted by 1 to 3 substituents independently selected from alkyl, alkoxy, cycloalkyl, cyano, and aminomethyl, $y^b$-$C_6H_4S$-; $y^b$-$C_6H_4O$-,

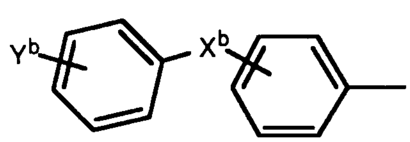

,

α-naphthyl, β-naphthyl, $H_2N(CH_2)_m{}^a$- or diphenylmethyl;

$R^{2b}$ is alkyl, alkyl-$S(O)_{0-2}(CH_2)_q{}^b$- , $R^{5b}(CH_2)_k{}^b$-$S(O)_{0-2}(CH_2)_q{}^b$ , alkyl-$O(CH_2)_q{}^b$- , $R^{5b}(CH_2)_k{}^b$-$O(CH_2)_q{}^b$- , $R^{5b}(CH_2)_q{}^b$- , $H_2N(CH_2)_q{}^b$- , cycloalkyl$(CH_2)_k{}^b$- , $R^{13b}CONH(CH_2)_q{}^b$- , $R^{13b}NHCO(CH_2)_q{}^b$- or $R^{6b}OCO(CH_2)_q{}^b$- ;

$R^{3b}$ is -$OR^{7b}$, -$NR^{7b}R^{8b}$,

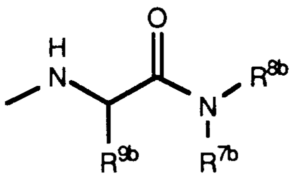 or 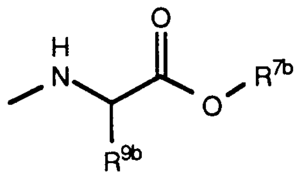 ;

$R^{4b}$ and $R^{13b}$ are independently hydrogen, alkyl or $Y^{1b}$-$C_6H_4$- ;

$R^{5b}$ is $Y^{2b}$-$C_6H_4$- , $Y^{2b}$-$C_6H_4S$- , $Y^{2b}$-$C_6H_4O$- , $\alpha$-naphthyl, $\beta$-naphthyl or

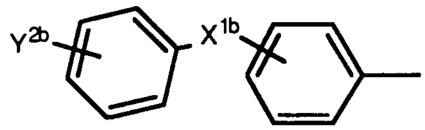

provided that when $R^{5b}$ is $Y^{2b}$-$C_6H_4S$- or $Y^{2b}$-$C_6H_4O$- , $k^b$ is 2 or 3;

$R^{6b}$, $R^{7b}$ and $R^{8b}$ are independently H, alkyl, hydroxyalkyl, alkoxyalkyl, aminoalkyl, alkylaminoalkyl, dialkylaminoalkyl, phenylalkyl, naphthylalkyl, $Y^b$-phenylalkyl or $Y^b$-naphthylalkyl;

$R^{9b}$ is hydrogen alkyl, carboxyalkyl, mercaptoalkyl, alkylthioalkyl, aminoalkyl, hydroxyalkyl, phenylalkyl, hydroxyphenylalkyl, guanidinoalkyl or carbamoylalkyl;

$n^b$ is 0-2;

$m^b$ and $k^b$ are independently 0-3;

$q^b$ is 1-4;

$X^b$ and $X^{1b}$ are independently a bond, -O- , -S- or $-CH_2-$ ;

$Q^b$ is hydrogen or $R^{10b}CO$- ;

$R^{10b}$ is alkyl, hydroxyalkyl, alkoxyalkyl, dialkylaminoalkyl, $Y^{2b}$-$C_6H_4$-alkyl, alkoxy, $Y^{2b}$-$C_6H_4$- or naphthyl;

$Y^b$, $Y^{1b}$, $Y^{2b}$ and $Y^{2b}$ independently represent 1 to 3 substituents selected from H, alkyl, cycloalkyl, alkoxy, OH, F, Cl, Br, CN, $-CH_2NH_2$, -COOH, -COOalkyl, $-CONH_2$ and phenyl;

$R^{1ab}$ is $Y^b$-$C_6H_4$- , $Y^b$-$C_6H_4S$- , $Y^b$-$C_6H_4O$- ,

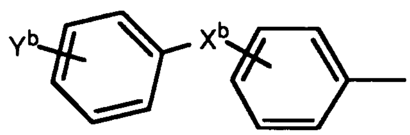 ,

$\alpha$-naphthyl, $\beta$-naphthyl, $H_2N(CH_2)m^b$- or diphenylmethyl;

$R^{2ab}$ is $R^{5ab}(CH)_k{}^bS(O)_{0-2}(CH_2)_q{}^b$- , $R^{5ab}(CH)_q{}^b$- or cycloalkyl-$(CH_2)_k{}^b$- , and when $R^{3b}$ is $-NR^{7b}R^{8b}$,

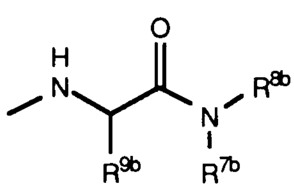 or 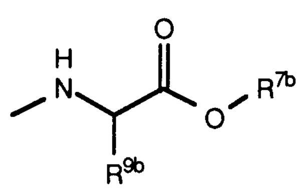 ,

$R^{2ab}$ may also be $R^{13b}CONH(CH_2)_q{}^b$- , $R^{13b}NHCO(CH_2)_q{}^b$-or $R^{6b}OCO(CH_2)_q{}^b$- ;

$R^{3ab}$ is $-OR^{11b}$, $-NR^{11b}R^{12b}$,

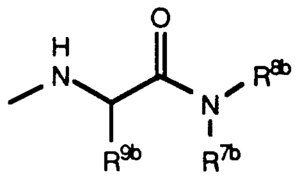 or 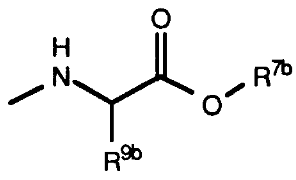 ;

$R^{5ab}$ is $Y^{2b}$-$C_6H_4$- provided $Y^{2b}$ is not H or OH, $Y^{2b}$-$C_6H_4S$- , $Y^{2b}$-$C_6H_4O$- , $\alpha$-naphthyl, $\beta$-naphthyl or

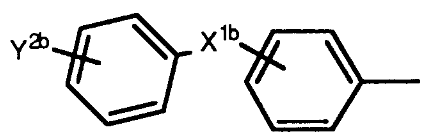 ,

provided that when $R^{5ab}$ is $Y^{2b}$-$C_6H_4S$- , $Y^{2b}$-$C_6H_4O$- , $k^b$ is 2 or 3;

$R^{11b}$ is hydroxyalkyl, substituted phenylalkyl wherein the phenyl group is substituted by 1 or more groups selected from alkyl, alkoxy, cycloalkyl and cyano;

$R^{12b}$ is H or selected from the same group as $R^{11b}$;

wherein the term alkyl or each alkyl portion has 1-6 carbon atoms and cycloalkyl is $C_3$-$C_6$; or pharmaceutically acceptable addition salts thereof;

mercaptoacyl amino acids disclosed in U.S. patent 4,929,641, having the formula

$Q^cS$-$CH_2$-$CH(-(CH_2)_{n^c}$-$R^{1c})$-C(O)-NH-CH($R^{2c}$)-C(O)-$R^{3c}$

wherein $R^{1c}$ is phenyl substituted by alkyl, $R^{2c}$ is alkyl-$S(O)_{0-2}(CH_2)_{q^c}$- , $R^{3c}$ is $OR^{7c}$ wherein $R^{7c}$ is hydrogen or lower alkyl, $Q^c$ is hydrogen or $R^{10c}$CO- wherein $R^{10c}$ is alkyl, $n^c$ is 0-2 and $q^c$ is 1-4; wherein preferred compounds are N-[2-acetylthiomethyl-3-(2-methyl-phenyl)propionyl]-methionine ethyl ester and N-[2-mercaptomethyl-3-(2-methylphenyl)propioyl]-methionine;

mercaptoacyl amino acids disclosed in PCT Publication WO90/12003, having the formula

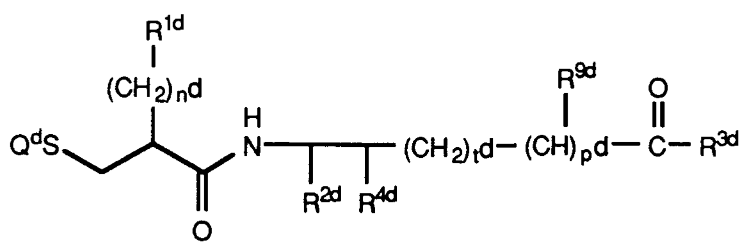

wherein:

| | |
|---|---|
| $Q^d$ | is hydrogen or $R^{7d}$CO- ; |
| $R^{1d}$ | is lower alkyl, cyclolower alkyl, aryl or heteroaryl; |
| $R^{2d}$ | is hydrogen; lower alkyl; cyclolower alkyl; lower alkyl substituted with hydroxy, lower alkoxy mercapto, lower alkylthio, aryl or heteroaryl; aryl; or heteroaryl; |
| $R^{3d}$ | is $-OR^{5d}$ or $-NR^{5d}R^{6d}$; |
| $R^{4d}$ and $R^{9d}$ | are independently $-(CH_2)_{q^d}R^{8d}$, provided that when $R^{4d}$ and $R^{9d}$ are both hydrogen, $R^{2d}$ is biphenyl, phenoxyphenyl, phenylthiophenyl, naphthyl, heteroaryl or lower alkyl substituted with hydroxy, lower alkoxy, mercapto or lower alkylthio; |
| $R^{5d}$ and $R^{6d}$ | are independently selected from the group consisting of hydrogen, lower alkyl, hydroxy lower alkyl, lower alkoxy lower alkyl and aryl lower alkyl, or $R^{5d}$ and $R^{6d}$ together with the nitrogen to which they are attached form a 5-7 membered ring; |
| $R^{7d}$ | is hydrogen, lower alkyl or aryl; |
| $R^{8d}$ | is hydrogen, hydroxy, lower alkoxy, mercapto, lower alkylthio, aryl or heteroaryl; |

$n^d$ is 1 or 2;
$p^d$ is 0 or 1
$q^d$ is 0, 1 or 2;
$t^d$ is 0 or 1;

and pharmaceutically acceptable salts thereof; wherein preferred compounds are N-[2(S)-mercaptomethyl-3-(2-methylphenyl)propanoyl]-(S)-isoserine and N-(S)-[3-mercapto-2-(2-methylphenyl)propionyl]-(S)-2-methoxy-$\beta$-alanine;

carboxyalkyl dipeptides disclosed in PCT Publication WO91/05796, having the formula

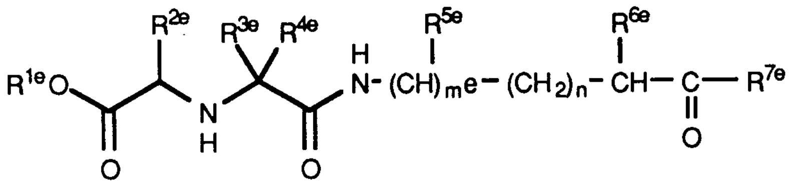

wherein:

$R^{1e}$ is H, alkyl, arylalkyl, or aryl;
$R^{2e}$ is H, alkyl, alkenyl or alkynyl, wherein the alkyl portion is substituted with 0-3 substituents independently selected from the group consisting of hydroxy, alkoxy, alkoxyalkoxy, alkylthio, aryl, alkoxyalkylthio, arylalkoxy and arylalkylthio;
$R^{3e}$ and $R^{4e}$ are independently alkyl orarylalkyl; or $R^{3e}$ and $R^{4e}$ together with the carbon to which they are attached form a 5-, 6- or 7-membered ring wherein said ring comprises 0 to 1 heteroatoms selected from the group consisting of sulfur and oxygen, wherein said ring is unsubstituted or is substituted on a carbon atom ring member by an alkyl or aryl group, or wherein said ring is substituted by a fused benzene ring;
$R^{5e}$ is H, alkyl, alkoxyalkyl, alkylthioalkyl, aryl, arylalkyl, heteroaryl, heteroarylalkyl, arylalkoxyalkyl or arylalkylthioalkyl;
$R^{6e}$ is H, hydroxy, alkoxy, alkyl, alkoxyalkyl, alkylthioalkyl, arylalkoxyalkyl, arylalkylthioalkyl, aryl, heteroaryl, arylalkyl or heteroarylalkyl;
$R^{7e}$ is hydroxy, alkoxy, aryloxy, arylalkoxy, amino, alkylamino or dialkylamino;
$m^e$ is 0 or 1;
$n^e$ is 0, 1, 2 or 3;

or a pharmaceutically acceptable salt thereof; wherein preferred compounds are N-[1-[[1(S)-benzyloxycarbonyl-3-phenylpropyl]amino]-cyclopentylcarbonyl]-(S)-isoserine and N-[1-[[1(S)-carbonyl-3-phenylpropyl]amino]-cyclopentylcarbonyl]-(S)-isoserine;

disulfide derivatives of mercaptoacyl amino acids disclosed in PCT Publication WO91/17980, having the formulae

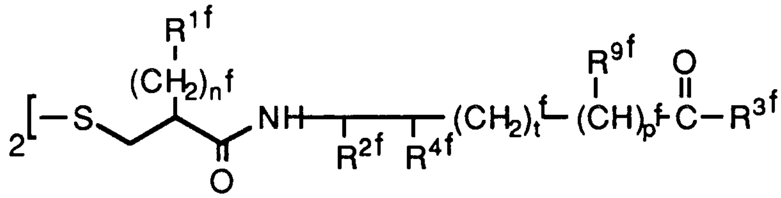

If

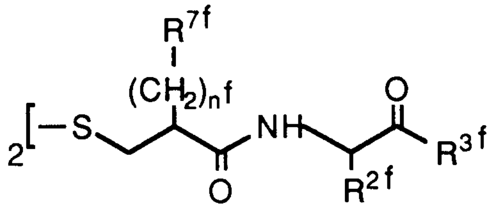

IIf

wherein:

$R^{1f}$ is lower alkyl, cyclolower alkyl, aryl or heteroaryl;

$R^{2f}$ is hydrogen; loweralkyl; cyclolower alkyl; lower alkyl substituted with hydroxy, lower alkoxy, mercapto, lower alkylthio, aryl, heteroaryl, aralkyloxy or aralkylthio; aryl; or heteroaryl;

$R^{3f}$ is $-QR^{5f}$ or $-NR^{5f}R^{6f}$;

$R^{4f}$ and $R^{9f}$ are independently $-(CH_2)_q{}^fR^{8f}$;

$R^{5f}$ and $R^{6f}$ are independently selected from the group consisting of hydrogen, lower alkyl, hydroxy lower alkyl, lower alkoxy lower alkyl and aryl loweralkyl, or $R^{5f}$ and $R^{6f}$ together with the nitrogen to shich they are attached form a 5-7 membered ring;

$p^{7f}$ is phenyl substituted by 1 to 3 substituents selected from the group consisting of lower alkyl, lower alkoxy, cycloalkyl, halo, cyano and aminomethyl;

$R^{8f}$ is hydrogen, hydroxy, lower alkoxy, mercapto, lower alkylthio, aryl or heteroaryl;

$n^f$ is 1 or 2;

$p^f$ is 0 or 1;

$q^f$ is 0, 1, or 2;

$t^f$ is 0 or 1;

or a pharmaceutically acceptable salt thereof; wherein preferred compounds are 1,1'-[dithiobis-[2(S)-(2-methylbenzyl)-1-oxo-3,1-propanediyl]]-bis-(S)-isoserine and 1,1'-[dithiobis-[2(S)-(2-methylbenzyl)-1-oxo-3,1-propanediyl]]-bis-(S)-methionine;

mercaptoacyl amino acids disclosed in PCT publication WO90/02117, having the formula

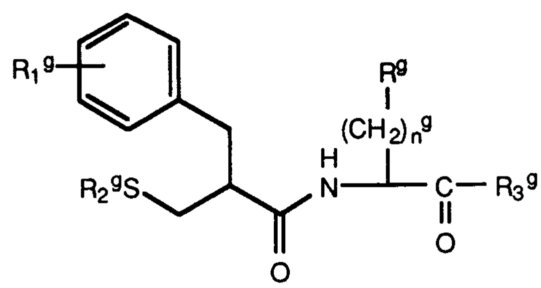

wherein:

R9 is a group of the formulae

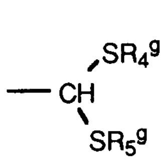 , 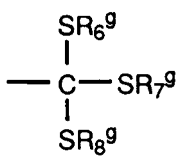 or 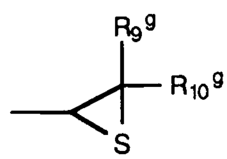 ;

$R_1{}^g$ is 1-3 substituents selected from the group consisting of hydrogen, halogen, lower alkyl, cycloalkyl, lower alkoxy, OH, aryl, aryloxy, CN, $-CH_2NH_2$, -COOH, lower alkoxycarbonyl and $-CONH_2$;

$R_2{}^g$ is H, $-COR_{11}{}^g$ or $-CH_2OCOR_{11}{}^g$;

$R_3{}^g$ is $-OR_{12}{}^g$, $-NR_{12}{}^gR_{13}{}^g$ or $-OCH(R_{14}{}^g)CONR_{12}{}^gR_{13}{}^g$;

$R_4{}^g$, $R_5{}^g$, $R_6{}^g$, $R_7{}^g$, $R_8{}^g$, $R_9{}^g$ and $R_{10}{}^g$ are each independently lower alkyl; or aralkyl or cycloalkylalkyl, wherein the aryl or cycloalkyl ring is optionally substituted by 1-3 substituents selected from lower alkyl, OH, halogen, lower alkoxy and $NH_2$; or $R_4{}^g$ and $R_5{}^g$ together with the carbon to which they are attached comprise a 5-, 6- or 7-membered ring;

$R_{11}{}^g$ is lower alkyl, aryl or arylmethyl;

$R_{12}{}^g$ and $R_{13}{}^g$ are each independently hydrogen; lower alkyl, optionally substituted by 1-2 OH or lower alkoxy groups; lower alkoxyalkoxy; halogen; lower haloalkoxy; $NH_2$; alkylamino; dialkylamino; Ar;

or Het; or $R_{12}^{g}$ and $R_{13}^{g}$ together with the nitrogen to which they are attached comprise a 5-, 6- or7-membered ring, wherein said ring may contain ring members selected from the group consisting of -N- , -N(alkyl)- or -O- , and wherein said ring is optionally substituted at a carbon atom ring member by an alkyl or OH substituent;

$Ar^{g}$ is an aryl group optionally substituted by 1-3 substituents selected from the group consisiting of lower alkyl, OH, halogen, lower alkoxy and $NH_2$;

$Het^{g}$ is a 5- or 6-membered saturated ring, wherein 1-2 ring memebers are oxygen atoms and wherein said ring is optionally substituted by 1-2 lower alkyl groups;

$R_{14}^{g}$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl or carbamoylalkyl;

$n^{g}$ is 0, 1, 2, 3 or 4;

and pharmaceutically acceptable addition salts thereof; wherein a preferred compound is N-(3-phenyl-2-(mercaptomethyl)-propionyl)-(S)-4-(methylmercapto)methionine;

mercaptoacyl amino acids disclosed in U.S. patent 4,879,309, having the formula

$R_1{}^{h}S\text{-}CH_2\text{-}CH(\text{-}(CH_2)_n{}^{h}\text{-}R_2{}^{h})\text{-}C(O)\text{-}NR_4{}^{h}\text{-}A^{h}\text{-}C(O)\text{-}R_3{}^{h}$

wherein:

$R_1{}^{h}$ is H or $R_5{}^{h}CO$-;

$R_2{}^{h}$ is $Y^{h}$-$C_6H_4$- , $Y^{h}$-$C_6H_4S$- , $Y^{h}$-$C_6H_4O$- , $Y^{h}$-$C_6H_4CH_2S$- , $Y^{h}$-$C_6H_4CH_2O$- , $\alpha$-naphthyl, $\beta$-naphthyl, furyl, thienyl, benzofuryl, benzothienyl, diphenylmethyl or

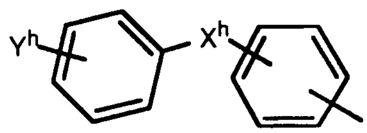

;

$R_3{}^{h}$ is $-OR_6{}^{h}$, $-NR_6{}^{h}R_7{}^{h}$ or

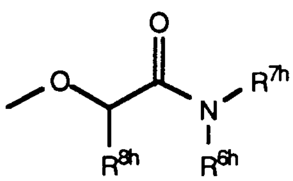

;

$R_4{}^{h}$ is hydrogen, lower alkyl or aryl lower alkyl;

$R_5{}^{h}$ is lower alkyl, hydroxylower alkyl, lower alkoxy lower alkyl, (di-lower alkyl) amino lower alkyl, $Y_1{}^{h}$-$C_6H_4$-lower alkyl, lower alkoxy, $Y_1{}^{h}$-$C_6H_4$- , naphthyl, fury, thienyl or pyridyl;

$R_6{}^{h}$ and $R_7{}^{h}$ are independently hydrogen, lower alkyl or substituted lower alkyl wherein the substituents are selected from the group consisting of 1 or 2 hydroxy groups, 1 or 2 lower alkoxy groups, lower alkoxy lower alkoxy, halogeno, halogeno lower alkoxy, amino, mono- or di-lower alkyl amino, heterocycloalkyl, lower alkyl heterocycloalkyl, aryl, substituted aryl wherein the substituents on aryl are 1-3 substituents selected from the group consisting of lower alkyl, hydroxy, halogeno, lower alkoxy and amino, and a 5-6 membered saturated ring comprising 1-2 oxygen atoms as ring members wherein the ring carbon atoms are substituted with 0-2 lower alkyl substituents; or $R_6{}^{h}$ and $R_7{}^{h}$ together with the nitrogen to which they are attached complete a 5-7 membered ring, wherein 0-1 of the 4-6 ring members comprising $R_6{}^{h}$ and $R_7{}^{h}$ is a

nitrogen atom, an alkyl substituted nitrogen atom or an oxygen atom, and wherein the ring is substituted on the ring carbon atoms with 0-3 substituents selected from the group consisting of alkyl and hydroxy;

$R_8{}^h$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl or carbamoylalkyl;

$n^h$ is 0-3;

$A^h$ is

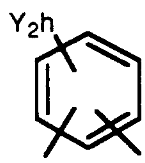 , 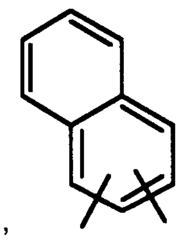 , 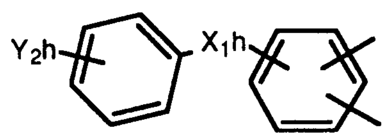 or 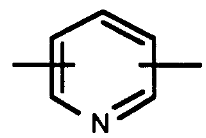 ;

$X^h$ and $X_1{}^h$ are independently a bond, -O- , -S- or $-CH_2-$ ;

$Y^h$, $Y_1{}^h$ and $Y_2{}^h$ are independently 1 to 3 substituents selected from the group consisting of hydrogen, lower alkyl, cyclolower alkyl, lower alkoxy, OH, F, Cl, Br, I, -CN, -COOH, -COO-lower alkyl, $-CH_2NH_2$, $-CONH_2$ and aryl;

and the pharmaceutically acceptable acid addition salts thereof; wherein preferred compounds are N-[2-acetylthiomethyi-3-phenyl-propionyl]-3-aminobenzoic acid and N-[2-mercaptomethyl-3-phenyl-propionyl]-3-aminobenzoic acid;

carboxyalkylcarbonyl amino acids disclosed in PCT Publication WO91/07386, having the formula

$R^{1i}O-C(O)-CH(R^{2i})-CH_2-C(R^{3i}R^{4i})-C(O)-NH-(CHR^{5i})_m{}^i-(CH_2)_n{}^i-CH(R^{6i})-C(O)-R^{7i}$

wherein:

$p^{1i}$ is H, alkyl, arylalkyl, aryl or aryloxyalkyl;

$R^{2i}$ is alkyl, alkenyl, alkynyl, alkoxy or alkylthio, wherein the alkyl portion is substituted with 0-3 substituents independently selected from the group consisting of hydroxy, alkoxy, alkoxyalkoxy, alkylthio, aryl, alkoxyalkylthio, arylalkoxy and arylalkylthio;

$R^{3i}$ and $R^{4i}$ are independently alkyl or arylalkyl; or $R^{3i}$ and $R^{4i}$ together with the carbon to which they are attached form a 5- , 6- or 7-membered ring wherein said ring comprises 0 to 1 heteroatoms selected from the group consisting of sulfur and oxygen, wherein said ring is unsubstituted or is substituted on a carbon atom ring member by an alkyl or aryl group, or wherein said ring is substituted by a fused benzene ring;

$p^{5i}$ is H, alkyl, alkoxyalkyl, alkylthioalkyl, aryl, arylalkyl, heteroaryl, heteroarylalkyl, arylalkoxyalkyl or arylalkylthioalkyl;

$R^{6i}$ is H, hydroxy, alkoxy, alkyl, arylalkoxy, alkoxyalkyl, alkylthioalkyl, arylalkoxyalkyl, arylalkylthioalkyl, aryl or heteroaryl;

$R^{7i}$ is hydroxy, alkoxy, aryloxy, arylalkoxy, amino, alkylamino or dialkylamino;

$m^i$ is 0 or 1;

$n^i$ is 0, 1, 2 or 3;

or a pharmaceutically acceptable salt thereof; wherein a preferred compound is N-[1-(2-carboxy-4-phenylbutyl)-cyclopentanecarbonyl]-(S)-isoserine;

mercaptocycloalkyl amino acids disclosed in PCT Publication WO91/09840, having the formula

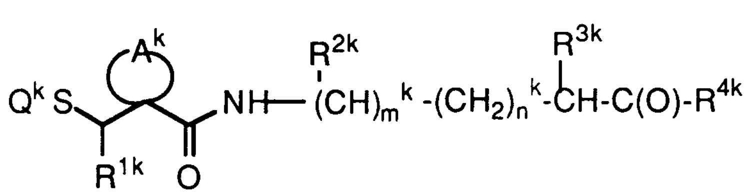

wherein:

$A^k$ is a 4-, 5- or 6-membered alkylene chain substituted with 1 to 3 substituents selected from the group consisting of hydrogen, hydroxy, alkyl or aryl; a 4-, 5- or 6-membered alkenylene chain, wherein 1 to 3 of the saturated carbon atoms are substituted as defined fort the alkylene chain; a 4-, 5- or 6-membered hetero-atom-containing chain comprising 2 to 5 carbon atoms and 1 or 2 hetero atoms selected from the group consisting of oxygen and sulfur, wherein when 2 heteroatoms are present, the heteroatoms are non-adjacent; or an alkylene or hetero-atom-containing chain as defined above wherein said alkylene or hetero-atom-containing chain is substituted with a fused benzene ring;

$Q^k$ is hydrogen or $R^{5k}CO$- ;

$R^{1k}$ is H, alkyl, arylalkyl or aryl;

$R^{2k}$ is H, alkyl, alkoxyalkyl, alkylthioalkyl, aryl, arylalkyl, heteroaryl, heteroarylalkyl, arylalkoxyalkyl, or arylalkylthioalkyl;

$R^{3k}$ is H, hydroxy, alkoxy, alkyl, arylalkoxy, alkoxyalkyl, alkylthioalkyl, arylalkoxyalkyl, arylalkylthioalkyl, aryl, heteroaryl, arylalkyl or heteroarylalkyl, or $R^{2k}$ and $R^{3k}$ form an alkylene chain of 1 to 5 carbon atoms, thereby, together with the carbons to which they are attached, completing a 5-, 6-, or 7-membered ring;

$R^{4k}$ is hydroxy, alkoxy, aryloxy, arylalkoxy, amino, alkylamino or dialkylamino;

$R^{5k}$ is alkyl or aryl;

$m^k$ is 0 or 1;

$n^k$ is 0, 1, 2 or 3;

or a pharmaceutically acceptable salt thereof; wherein a preferred compound is N-[1-(acetylthiomethyl)-cyclopentane-carbonyl]-(S)-methionine ethyl ester;

mercaptoacyl aminolactams disclosed in U.S.Patent 5,075,302, having the formula

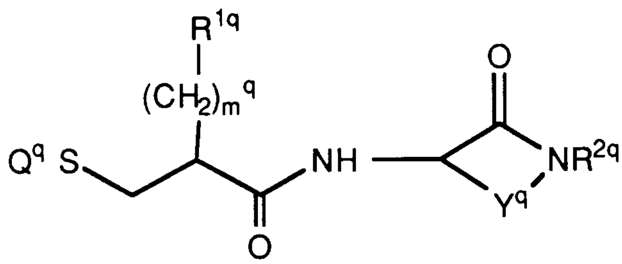

wherein:

$Y^q$ is $-(CHR^{5q})_{n^q}-(CR^{3q}R^{4q})-$ or $-(CR^{3q}R^{4q})_{p^q}X^q(CR^{3q}R^{4q})_{q^q}-$ , wherein two substituents selected from the group consisting of $R^{3q}$, $R^{4q}$ and $R^{5q}$, together with the carbons to which the substituents are attached, when the substituents are present on adjacent carbon atoms, can form a benzene, cyclopentane or cyclohexane ring;

$X^q$ is -O- , -S- ,-SO- or $-SO_2-$;

$Q^q$ is hydrogen or $R^{6q}CO$- ;

$m^q$ is 1 or 2;

$n^q$ is 1, 2, 3 or 4;

$p^q$ is 1 or 2;

$q^q$ is 2 or 3;

$R^{1q}$ is lower alkyl; aryl selected from the group consisting of phenyl, naphthyl, substituted phenyl and substituted naphthyl, wherein said substituted phenyl and substituted naphthyl groups are substituted with 1-3 substituents selected from the group consisting of lower alkyl, hydroxy, lower alkoxy, halo, trifluoromethyl, phenyl, phenoxy, and phenylthio; heteroaryl or substituted heteroaryl, wherein heteroaryl is selected from the group consisting of furanyl, thienyl, pyrrolyl and pyridyl, and wherein said substituted heteroaryl is substituted with 1-3 substituents selected from the group consisting of lower alkyl, hydroxy, lower alkoxy, halo, trifluoromethyl, phenyl, phenoxy, and phenylthio;

$R^{2q}$ is hydrogen, lower alkyl, hydroxylower alkyl, loweralkoxylower alkyl, aryllower alkyl, heteroaryllower alkyl, substituted-aryllower alkyl or substituted-heteroaryllower alkyl, wherein aryl, heteroaryl, substituted aryl and substituted heteroaryl are as defined above;

$p^{3q}$ and $R^{4q}$ are independently hydrogen, loweralkyl, aryllower alkyl, heteroaryllower alkyl,

substituted-aryllower alkyl or substituted-heteroaryllower alkyl, wherein aryl, heteroaryl, substituted aryl and substituted heteroaryl are as defined above;

$R^{5q}$ is hydrogen, loweralkyl, aryllower alkyl, heteroaryllower alkyl, hydroxy, lower alkoxy, mercapto, lower alkylthio, substituted-aryllower alkyl or substituted-heteroaryllower alkyl, wherein aryl, heteroaryl, substituted aryl and substituted heteroaryl are as defined above; and

$R^{6q}$ is lower alkyl, aryl or heteroaryl, wherein aryl and heteroaryl are as defined above;

wherein a preferred compound is 3(S)-[2-(acetylthiomethyl)-3-phenyl-propionyl]amino-ε-caprolactam;

glutaryl amino acids disclosed in U.S. 4,975,444, having the formula

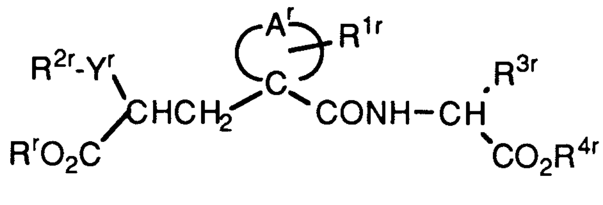

wherein:

$A^r$ completes a 5 or 6 membered carbocyclic ring which may be saturated or monounsaturated;

$R^{1r}$ is H or $(C_1$-$C_4)$alkyl;

$R^r$ and $R^{4r}$ are each independently H, $(C_1$-$C_6)$alkyl, $(C_3$-$C_7)$cycloalkyl, benzyl or an alternative biolabile ester-forming group;

$Y^r$ is either a direct bond or an alkylene group of from 1 to 6 carbon atoms which may be straight or branched chain;

$R^{2r}$ is H, aryl, heterocyclyl, $R^{6r}CONR^{5r}$- , $R^{7r}NR^{5r}CO$- , $R^{7r}NR^{5r}SO_2$- or $R^{8r}SONR^{5r}$- , with the proviso that $Y^r$ is not a direct bond when $R^{2r}$ is H, aryl or heterocyclyl;

$R^{3r}$ is agroup of the formula

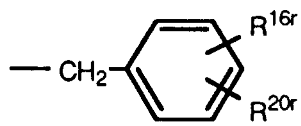 ; or

a group of the formula

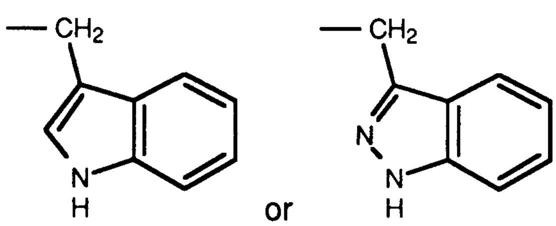

wherein said groups may optionally be substituted in the fused benzene ring by $(C_1$-$C_4)$alkyl, $(C_1$-$C_4)$alkoxy, OH, halo or $CF_3$;

$R^{5r}$ is H, $(C_1$-$C_6)$alkyl or aryl$(C_1$-$C_6)$alkyl;

$R^{6r}$ is aryl, heterocyclyl or a group of the formula

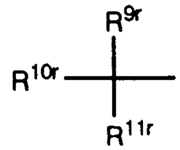
;

$R^{7r}$ is $(C_1-C_6)$alkyl, aryl, aryl$(C_1-C_6)$alkyl, heterocyclyl, heterocyclyl$(C_1-C_6)$alkyl or a group of the formula

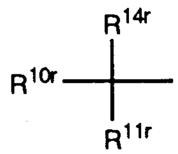
;

$R^{8r}$ is $(C_1-C_6)$alkyl, aryl, aryl$(C_1-C_6)$alkyl, heterocyclyl or heterocyclyl$(C_1-C_6)$alkyl;

$R^{9r}$ is H, OH, $(C_1-C_6)$alkoxy, $(C_1-C_6)$alkyl, hydroxy$(C_1-C_6)$alkyl, aryl, aryl$(C_2-C_6)$heterocyclyl, heterocyclyl$(C_1-C_6)$alkyl, $R^{12r}CONH-$ , $R^{12r}SO_2NH-$ or $(R^{13r})_2N-$ ;

$R^{10r}$ and $R^{11r}$ are each independently H or alkyl; or $R^{10r}$ is H and $R^{11r}$ is amino, $(C_1-C_6)$alkyl, imidazolylmethyl, aryl, aryl$(C_1- C_6)$alkyl, hydroxy$(C_1-C_6)$alkyl or methylthio$(C_1-C_6)$alkyl; or the two groups $R^{10r}$ and $R^{11r}$ are joined together to form, with the carbon atom to which they are attached, a 3 to 6 membered carbocyclic ring or a ring which may optionally be substituted by amino, $(C_2-C_4)$alkanoyl or aroyl; or a pyrrolidine or piperidine ring which is substituted by amino, $(C_2-C_4)$alkyl or aroyl; amino;

$R^{12r}$ is $(C_1-C_6)$alkyl, $(C_3-C_7)$cycloalkyl, aryl, aryl$(C_1-C_6)$alkyl, heterocyclyl or heterocyclyl$(C_1-C_6)$alkyl;

$R^{13r}$ is H, $(C_1-C_6)$alkyl, aryl or the two groups $R^{13r}$ are taken together to form, with the nitrogen to which they are attached, a pyrrolidinyl, piperidino, morpholino, piperazinyl or N-$(C_1-C_6)$alkyl-piperazinyl group;

$R^{14r}$ is $(R^{13r})_2NCO-$ , $R^{12r}OCH_2-$ or $R^{15r}OCO-$ ;

$R^{15r}$ is $(C_1-C_6)$alkyl, $(C_3-C_7)$cycloalkyl or aryl$(C_1-C_6)$alkyl;

$R^{16r}$ is H, halo, 4-hydroxy, 4-$(C_1-C_6$ alkoxy), 4-$(C_3-C_7$ cycloalkoxy), 4-$(C_2-C_6$ alkenyloxy), 4-[$(C_1-C_6$ alkoxy)carbonyloxy], 4-[$(C_3-C_7$ cycloalkoxy)carbonyloxy] or 3-$(C_1-C_4$ alkyl)-$SO_2NH-$;

$R^{20r}$ is H, $(C_1-C_4)$alkyl, $(C_1-C_4)$alkoxy$(C_2-C_6)$alkanoyl or halo;

and pharmaceutically acceptable salts thereof and bioprecursors therefor;

glutaryl amino acids disclosed in European Patent Application 274,234, having the formula

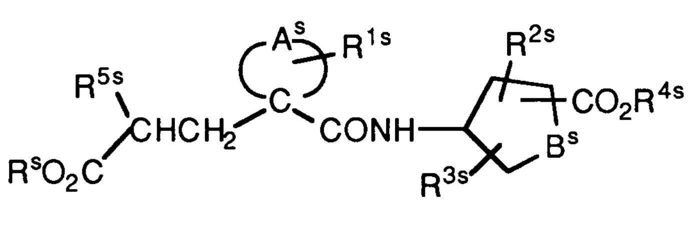

wherein:

$A^s$ completes a 4 to 7 membered carbocyclic ring which may be saturated or mono-unsaturated and which may optionally be fused to afurther saturated or unsaturated 5 or 6 membered carbocyclic ring;

$B^s$ is $(CH_2)m^s$ wherein $m^s$ is an integer of from 1 to 3;

$R^s$ and $R^{4s}$ are each independently H, alkyl, benzyl or an alternative biolabile ester-forming group;

$R^{1s}$ is H or alkyl;

$R^{2s}$ and $R^{3s}$ are each independently H, OH, alkyl or alkoxy;

| | |
|---|---|
| $R^{5s}$ | is alkyl, alkenyl, alkynyl, arylalkynyl, cycloalkyl, cycloalkenyl, alkoxy, $-NR^{6s}R^{7s}$, $-NR^{8s}COR^{9s}$, $-NR^{8s}SO_2R^{9s}$ ora saturated heterocyclic group; or alkyl substituted by one or more substituents chosen from halo, hydroxy, alkoxy, hydroxyalkoxy, alkoxyalkoxy, cycloalkyl, cycloalkenyl, aryl, aryloxy, arlyoxyalkoxy, heterocyclyloxy, $-NR^{6s}R^{7s}$, $-NR^{8s}COR^{9s}$, $-NR^{8s}SO_2R^{9s}$, $-CONR^{6s}R^{7s}$, -SH, $-S(O)_p{}^sR^{10s}$, $-COR^{11s}$ or $-CO_2R^{12s}$; |
| $R^{6s}$ and $R^{7s}$ | are each independently H, alkyl, cycloalkyl (optionally substituted by hydroxy or alkoxy), aryl, arylalkyl, alkoxyalkyl or heterocyclyl; or the two groups $R^{6s}$ and $R^{7s}$ are taken together with the nitrogen to which they are attached to form a pyrrolidinyl, piperidino, morpholino, piperazinyl or N-alkyl-piperazinyl group; |
| $R^{8s}$ | is H or alkyl; |
| $R^{9s}$ | is alkyl, $CF_3$, aryl, aryl, alkyl, arylalkoxy, heterocyclyl, alkoxy or $-NR^{6s}R^{7s}$ wherein $R^{6s}$ and $R^{7s}$ are as previously defined; |
| $R^{10s}$ | is alkyl, aryl, heterocyclyl, or $-NR^{6s}R^{7s}$ wherein $R^{6s}$ and $R^{7s}$ are as previously defined; |
| $R^{11s}$ | is alkyl, cycloalkyl, aryl or heterocyclyl; |
| $R^{12s}$ | is H or alkyl; |
| $p^s$ | is 0, 1 or 2; |

and pharmaceutically acceptable salts thereof and bioprecursors therefor; and

glutaryl amino acids disclosed in European Patent Application 343,911, having the formula

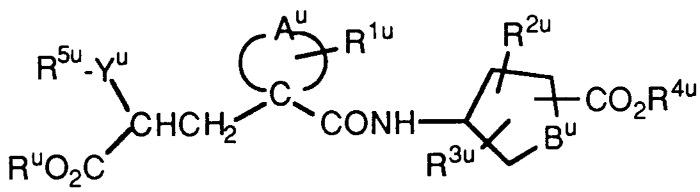

wherein:

| | |
|---|---|
| $A^u$ | completes a 4 to 7 membered carbocyclic ring which may be saturated or mono-unsaturated and which may optionally be fused to a further saturated or unsaturated 5 or 6 membered carbocyclic ring; |
| $B^u$ | is $(CH_2)m^u$ wherein $m^u$ is an integer of from 1 to 3; |

each of $R^u$ and $R^{4u}$ is independently H, alkyl, benzyl or an alternative biolabile ester-forming group;

| | |
|---|---|
| $R^{1u}$ | is H or alkyl; |
| $R^{2u}$ and $R^{3u}$ | are each independently H, OH, alkyl or alkoxy, or $R^{2u}$ and $R^{3u}$ are linked together and are $(CH_2)_r{}^u$ wherein $r^u$ is an integer from 1 to 4; |
| $Y^u$ | is an optional alkylene group of from 1 to 6 carbon atoms which may be straight or branched-chain; |
| $R^{5u}$ | is $R^{6u}CONR^{9u}$-, $R^{6u}SO_2NR^{9u}$-, $R^{6u}CO_2$-, $R^{6u}CO$-, $R^{6u}SO_q{}^u$-, $R^{7u}NR^{9u}SO_2$-, or $R^{7u}OCO$-; |
| $R^{6u}$ | is a group of the formula |

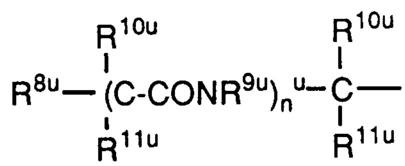

$R^{7u}$ is a group of the formula

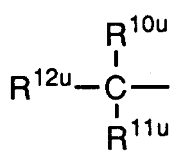

$R^{9u}$ is H, alkyl, aryl, cycloalkyl, heterocyclyl, arylalkyl, or hererocyclylalkyl;

| | |
|---|---|
| $R^{8u}$ | is $R^{9u}CONR^{9u}$-, $R^{9u}SO_2NR^{9u}$-, $R^{13u}R^{14u}N$-$(CH_2)_p{}^u$-, or $R^{9u}O$-, wherein each $R^{9u}$ is as previously defined; |
| $R^{10u}$ and $R^{11u}$ | are each independently H or alkyl; or $R^{10u}$ is H and $R^{11u}$ is alkyl which is substituted by OH, SH, $SCH_3$, $NH_2$, arylalkyl-OCONH-, $NH_2CO$-, $CO_2H$, guanidino, aryl or heterocyclyl; or the two groups $R^{10u}$ and $R^{11u}$ are joined together to form, with the carbon atom to which they are attached, a 5 or 6 membered carbocyclic ring which may be saturated or mono-unsaturated and which may optionally be substituted by alkyl or fused to a further 5 or 6 membered saturated or unsaturated carbocyclic ring; or $R^{10u}$ is H, $n^u$ is 0 and $R^{8u}$ and $R^{11u}$ are linked to form a 2-(N-$COR^{9u}$-4-aminopyrrolidinyl) group; |
| $R^{12u}$ | is $R^{13u}R^{14u}NCO$-, $R^{9u}OCH_2$- or heterocyclyl, wherein $R^{9u}$ is as previously defined; |
| $R^{13u}$ and $R^{14u}$ | are each independently H, alkyl, cycloalkyl, aryl, arylalkyl, alkoxyalkyl, aminoalkyl, heterocyclyl or heterocyclylalkyl; or the two groups $R^{13u}$ and $R^{14u}$ are taken together to form, with the nitrogen to which they are attached, a pyrrolidinyl, piperidino, morpholino, piperazinyl, N-alkylpiperazinyl, pyrrolyl, imidazolyl, pyrazolyl or triazolyl group; |
| $n^u$ | is 0 or 1; |
| $p^u$ | is 0 or an integer of from 1 to 6; |
| $q^u$ | is 0, 1 or 2; |

and pharmaceutically acceptable salts thereof and bioprecursors therefor.

The above descriptions of NEP inhibitors suitable for use in the present invention were taken from the noted patents or applications. Reference should be made to such patents and applications for their full disclosures of such classes and specific compounds within those classes, and as to any typographical errors or the like which may have occurred in transcription. Also, in describing such suitable NEP inhibitors, the superscript letters a-i, k, q-s and u were included to distinguish among the various classes of compounds and the variable substituent groups thereof.

Other suitable NEP inhibitors include SQ 28603 (N-[2-(mercaptomethyl)-1-oxo-3-phenylpropyl]-$\beta$-alanine), disclosed in South African Patent Application 84/0670; UK 69578 (cis-4-[[[1-[2-carboxy-3-(2-methoxyethoxy)propyl]-cyclopentyl]carbonyl]amino]-cyclohexanecarboxylic acid) and its active enantiomer(s); thiorphan and its enantiomers; retro-thiorphan; phosphoramidon; and SQ 29072 (7-[[2-(mercaptomethyl)-1-oxo-3-phenylpropyl]amino]-heptanoic acid). Also suitable for use are any pro-drug forms of the above-listed NEP inhibitors, e.g., compounds in which one or more carboxylic acid groups are esterified.

The effectiveness of NEP inhibitors in treating immunosuppressant-induced nephrotoxicity in an animal model can be demonstrated by modifying the procedure described in Capasso, et al by administering an NEP inhibitor in place of an ANP and measuring the resultant glomerular filtration rate, urine output and sodium excretion. The effectiveness of NEP inhibitors in preventing immunosuppression therapy-induced nephrotoxicity can be demonstrated by administering an NEP inhibitor from the start of immunosuppression therapy and monitoring the glomerular filtration rate, urine output and sodium excretion to show that there are no decreases in those rates.

For treatment of nephrotoxicity induced by immunosuppressive therapy, the method of this invention comprises administering an NEP inhibitor, preferably in pharmaceutical dosage form, to a mammal in need of such treatment, either after immunosuppression therapy is completed or during its course, whenever nephrotoxicity is detected. For prevention of nephrotoxicity, the method of this invention comprises administering an NEP inhibitor, preferably in pharmaceutical form, at substantially the same time as an immunosuppressant such as an immunosuppressant cyclosporin or FK-506 is administered. That is, a patient can undergo parallel courses of treatment with the two different drugs; simultaneous administration of dosage forms is not required. The NEP inhibitor and the immunosuppressant, when used concurrently, can be administered in combination in the same dosage unit or can be administered in separate dosage forms, either at the same time or on different dosing schedules, as necessary.

A variety of pharmaceutical dosage forms are suitable for NEP administration, preferably for oral or parenteral administration, although mechanical delivery systems such as transdermal dosage forms are also contemplated. Since the methods of this invention can comprise administering two different drugs, any suitable combination of dosage forms can be used, e.g. oral NEP inhibitor/ injectable immunosuppressant or injectable NEP inhibitor/ oral immnuosuppressant.

The typical daily dosage of the NEP inhibitor for this invention for treatment or prevention of nephrotoxicity is about 0.3 mg/kg to about 100 mg/kg of mammalian weight per day administered in single or divided doses. The exact dose of any NEP inhibitor to be administered is determined by the attending clinician and is dependent on the potency of the compound administered, the age, weight, condition and response of the patient.

Generally, in treating humans suffering from immunosuppression therapy-induced nephrotoxicity or in preventing such nephrotoxicity, the NEP inhibitors of this invention can be administered in dosage ranges of about 10 to about 500 mg NEP inhibitor per dose given 1 to 4 times a day.

Cyclosporin can be administered orally or parenterally and is usually administered in a single daily dose of 14-18 mg/kg, with the dosage level being gradually decreased to a maintenance dose of 5-10 mg/kg per day. It is contemplated that FK-506 can be administered at similar or lower dosage levels.

Typical oral formulations for compounds used in this invention include tablets, capsules, syrups, elixirs and suspensions. Typical injectable formulations for compounds used in this invention include solutions and suspensions.

The typical pharmaceutically acceptable carriers for use in the formulations described above are exemplified by: sugars such as lactose, sucrose, mannitol and sorbitol; starches such as cornstarch, tapioca starch and potato starch; cellulose and derivatives such as sodium carboxymethyl cellulose, ethyl cellulose and methyl cellulose; calcium phosphates such as dicalcium phosphate and tricalcium phosphate; sodium sulfate; calcium sulfate; polyvinylpyrrolidone; polyvinyl alcohol; stearic acid; alkaline earth metal stearates such as magnesium stearate and calcium stearate; stearic acid; vegetable oils such as peanut oil, cottonseed oil, sesame oil, olive oil and corn oil; non-ionic, cationic and anionic surtactants; ethylene glycol polymers; betacyclodextrin; fatty alcohols; and hydrolyzed cereal solids, as well as other non-toxic compatible fillers, binders, disintegrants, buffers, preservatives, antioxidants, lubricants, flavoring agents, and the like commonly used in pharmaceutical formulations.

Since the present invention relates to methods of treating or preventing nephrotoxicity with a combination of active ingredients, i.e. an NEP inhibitor and an immunosuppressant, wherein said active ingredients may be administered separately, the invention also relates to combining separate pharmaceutical compositions in kit form. That is, a kit which combines two separate units, an NEP inhibitor pharmaceutical composition and an immunosuppressant composition (particularly an immunosuppressant cyclosporin or an FK-506 composition), in one package is contemplated. The kit form is particularly advantageous when the separate components must be administered in different dosage forms (e.g. oral and parenteral) or are administered at different dosage intervals.

## Claims

1. A pharmaceutical composition comprising an amount of a neutral endopeptidase inhibitor effective to treat or prevent nephrotoxicity induced by immunosuppressant therapy in a pharmaceutically acceptable carrier.

2. A pharmaceutical composition comprising a combination of an effective amount of an immunosuppressant and amount of a neutral endopeptidase inhibitor effective to treat or prevent nephrotoxicity induced by said immunosuppressant in a pharmaceutically acceptable carrier.

3. A composition of claim 2 wherein the immunosuppressant is an immunosuppressant cyclosporin or FK-506

4. A composition of claims 1, 2 or 3 wherein the neutral endopeptidase inhibitor is selected from the group consisting of:
N-[N-[(L)-[1-[(2,2-dimethyl-1,3-dioxolan-4-yl)-methoxy]carbonyl]-2-phenylethyl]-L-phenylalanyl]-$\beta$-alanine;
N-[N-[(L)-1-carboxy-2-phenylethyl]-L-phenylalanyl]-$\beta$-alanine;
N-[2-acetylthiomethyl-3-(2-methyl-phenyl)propionyl]-methionine ethyl ester;
N-[2-mercaptomethyl-3-(2-methylphenyl)propioyl]-methionine;
N-[2(S)-mercaptomethyl-3-(2-methylphenyl)propanoyl]-(S)-isoserine;
N-(S)-[3-mercapto-2-(2-methylphenyl)propionyl]-(S)-2-methoxy-$\beta$-alanine;
N-[1-[[1(S)-benzyloxycarbonyl-3-phenylpropyl]amino]cyclopentylcarbonyl]-(S)-isoserine;
N-[1-[[1(S)-carbonyl-3-phenylpropyl]amino]-cyclopentylcarbonyl]-(S)-isoserine;
1,1'-[dithiobis-[2(S)-(2-methylbenzyl)-1-oxo-3,1-propanediyl]]-bis-(S)-isoserine;
1,1'-[dithiobis-[2(S)-(2-methylbenzyl)-1-oxo-3,1-propanediyl]]-bis-(S)-methionine;
N-(3-phenyl-2-(mercaptomethyl)-propionyl)-(S)-4-(methylmercapto)methionine;
N-[2-acetylthiomethyl-3-phenyl-propionyl]-3-aminobenzoic acid;
N-[2-mercaptomethyl-3-phenyl-propionyl]-3-aminobenzoic acid;
N-[1-(2-carboxy-4-phenylbutyl)-cyclopentanecarbonyl]-(S)-isoserine;

N-[1-(acetylthiomethyl)cyclopentane-carbonyl]-(S)-methionine ethyl ester;
3(S)-[2-(acetylthiomethyl)-3-phenyl-propionyl]amino-ϵ-caprolactam;
SQ 28603; UK 69578; thiorphan; retro-thiorphan; phosphoramidon; and SQ 29072.

**5.** The use of a neutral endopeptidase inhibitor for the preparation of a pharmaceutical composition useful in the treatment or prevention of nephrotoxicity induced by immunosuppression therapy wherein the immuno-suppression therapy comprises administering an immunosuppressant cyclosporin or FK-506.

**6.** The use according to claim 5, wherein the neutral endopeptidase inhibitor is selected from the group consisting of:
N-[N-[(L)-[1-[(2,2-dimethyl-1,3-dioxolan-4-yl)-methoxy]carbonyl]-2-phenylethyl]-L-phenylalanyl]-β-alanine;
N-[N-[(L)-1-carboxy-2-phenylethyl]-L-phenylalanyl]-β-alanine;
N-[2-acetylthiomethyl-3-(2-methyl-phenyl)propionyl]-methionine ethyl ester;
N-[2-mercaptomethyl-3-(2-methylphenyl)propioyl]-methionine;
N-[2(S)-mercaptomethyl-3-(2-methylphenyl)propanoyl]-(S)-isoserine;
N-(S)-[3-mercapto-2-(2-methylphenyl)propionyl]-(S)-2-methoxy-β-alanine;
N-[1-[[1(S)-benzyloxycarbonyl-3-phenylpropyl]amino]cyclopentylcarbonyl]-(S)-isoserine;
N-[1-[[1(S)-carbonyl-3-phenylpropyl]amino]-cyclopentylcarbonyl]-(S)-isoserine;
1,1'-[dithiobis-[2(S)-(2-methylbenzyl)-1-oxo-3,1-propanediyl]]-bis-(S)-isoserine;
1,1'-[dithiobis-[2(S)-(2-methylbenzyl)-1-oxo-3,1-propanediyl]]-bis-(S)-methionine;
N-(3-phenyl-2-(mercaptomethyl)-propionyl)-(S)-4-(methylmercapto)methionine;
N-[2-acetylthiomethyl-3-phenyl-propionyl]-3-aminobenzoic acid;
N-[2-mercaptomethyl-3-phenyl-propionyl]-3-aminobenzoic acid;
N-[1-(2-carboxy-4-phenylbutyl)-cyclopentanecarbonyl]-(S)-isoserine;
N-[1-(acetylthiomethyl)cyclopentane-carbonyl]-(S)-methionine ethyl ester;
3(S)-[2-(acetylthiomethyl)-3-phenyl-propionyl]amino-ϵ-caprolactam;
SQ 28603; UK 69578; thiorphan; retro-thiorphan; phosphoramidon; and SQ 29072.

**7.** A process forthe preparation of a pharmaceutical composition according to any of claims 1, 2, 3 or 4 which comprises mixing a neutral endopeptidase inhibitor, alone or in combination with an immunosuppressant, with a pharmaceutically acceptable carrier.

**8.** A method of treating or preventing nephrotoxicity induced by immunosuppression therapy comprising administering an effective amount of a neutral endopeptidase inhibitor to a mammal in need of such treatment.

**9.** A method of claim 8 wherein the immunosuppression therapy comprises administering an immunosuppressant cyclosporin or FK-506 and the neutral endopeptidase inhibitor is selected from the group consisting of:
N-[N-[(L)-[1-[(2,2-dimethyl-1,3-dioxolan-4-yl)-methoxy]carbonyl]-2-phenylethyl]-L-phenylalanyl]-β-alanine;
N-[N-[(L)-1-carboxy-2-phenylethyl]-L-phenylalanyl]-β-alanine;
N-[2-acetylthiomethyl-3-(2-methyl-phenyl)propionyl]-methionine ethyl ester;
N-[2-mercaptomethyl-3-(2-methylphenyl)propioyl]-methionine;
N-[2(S)-mercaptomethyl-3-(2-methylphenyl)propanoyl]-(S)-isoserine;
N-(S)-[3-mercapto-2-(2-methylphenyl)propionyl]-(S)-2-methoxy-β-alanine;
N-[1-[[1(S)-benzyloxycarbonyl-3-phenylpropyl]amino]cyclopentylcarbonyl]-(S)-isoserine;
N-[1-[[1(S)-carbonyl-3-phenylpropyl]amino]-cyclopentylcarbonyl]-(S)-isoserine;
1,1'-[dithiobis-[2(S)-(2-methylbenzyl)-1-oxo-3,1-propanediyl]]-bis-(S)-isoserine;
1,1'-[dithiobis-[2(S)-(2-methylbenzyl)-1-oxo-3,1-propanediyl]]-bis-(S)-methionine;
N-(3-phenyl-2-(mercaptomethyl)-propionyl)-(S)-4-(methylmercapto)methionine;
N-[2-acetylthiomethyl-3-phenyl-propionyl]-3-aminobenzoic acid;
N-[2-mercaptomethyl-3-phenyl-propionyl]-3-aminobenzoic acid;
N-[1-(2-carboxy-4-phenylbutyl)-cyclopentanecarbonyl]-(S)-isoserine;
N-[1-(acetylthiomethyl)cyclopentane-carbonyl]-(S)-methionine ethyl ester;
3(S)-[2-(acetylthiomethyl)-3-phenyl-propionyl]amino-ϵ-caprolactam;
SQ 28603; UK 69578; thiorphan; retro-thiorphan; phosphoramidon; and SQ 29072.

**10.** A kit comprising in separate containers in a single package pharmaceutical compositions for use in combination to treat or prevent nephrotoxicity induced by immunosuppressant therapy in a mammal which comprises in one container a pharmaceutical composition comprising an immunosuppressant and in a second container a pharmaceutical composition comprising a neutral endopeptidase inhibitor.

**11.** A kit of claim 10 wherein the immunosuppressant is an immunosuppressant cyclosporin or FK-506 and the neutral endopeptidase inhibitor is selected from the group consisting of:
N-[N-[(L)-[1-[(2,2-dimethyl-1,3-dioxolan-4-yl)-methoxy]carbonyl]-2-phenylethyl]-L-phenylalanyl]-β-alanine;
N-[N-[(L)-1-carboxy-2-phenylethyl]-L-phenylalanyl]-β-alanine;
N-[2-acetylthiomethyl-3-(2-methyl-phenyl)propionyl]-methionine ethyl ester;
N-[2-mercaptomethyl-3-(2-methylphenyl)propioyl]-methionine;
N-[2(S)-mercaptomethyl-3-(2-methylphenyl)propanoyl]-(S)-isoserine;
N-(S)-[3-mercapto-2-(2-methylphenyl)propionyl]-(S)-2-methoxy-β-alanine;
N-[1-[[1(S)-benzyloxycarbonyl-3-phenylpropyl]amino]cyclopentylcarbonyl]-(S)-isoserine;
N-[1-[[1(S)-carbonyl-3-phenylpropyl]amino]-cyclopentylcarbonyl]-(S)-isoserine;
1,1'-[dithiobis-[2(S)-(2-methylbenzyl)-1-oxo-3,1-propanediyl]]-bis-(S)-isoserine;
1,1'-[dithiobis-[2(S)-(2-methylbenzyl)-1-oxo-3,1-propanediyl]]-bis-(S)-methionine;
N-(3-phenyl-2-(mercaptomethyl)-propionyl)-(S)-4-(methylmercapto)methionine;
N-[2-acetylthiomethyl-3-phenyl-propionyl]-3-aminobenzoic acid;
N-[2-mercaptomethyl-3-phenyl-propionyl]-3-aminobenzoic acid;
N-[1-(2-carboxy-4-phenylbutyl)-cyclopentanecarbonyl]-(S)-isoserine;
N-[1-(acetylthiomethyl)cyclopentane-carbonyl]-(S)-methionine ethyl ester;
3(S)-[2-(acetylthiomethyl)-3-phenyl-propionyl]amino-ε-caprolactam;
SQ 28603; UK 69578; thiorphan; retro-thiorphan; phosphoramidon; and SQ 29072.

European Patent Office

# EUROPEAN SEARCH REPORT

Application Number

EP 92 10 6420

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,P | WO-A-9 203 410 (SCHERING CORPORATION) * page 5, lines 26-30; page 17, line 31 - page 18, line 2 * | 1-3 | A 61 K 37/64<br>C 07 K 15/00 |
| Y | EP-A-0 103 077 (SCHERING CORPORATION) * abstract * | 1-11 | |
| Y | EP-A-0 054 862 (SCHERING CORPORATION) * abstract *; & US - A - 4610816 (cat. D) | 1-11 | |
| A | GB-A-2 194 438 (MONSANTO) * the whole document *; & US - A - 4740499 (cat. D) | 1-11 | |
| A,D | EP-A-0 343 911 (PFIZER) * page 3, lines 4-16; page 8, line 46 - page 9, line 36 * | 1-11 | |
| A | FR-A-2 616 070 (SQUIBB & SONS) * page 7, line 16 - page 8, line 14; page 25, line 33 - page 26, line 7; pages 1-7 * | 1-11 | **TECHNICAL FIELDS SEARCHED (Int. Cl.5)** |
| A,P | WO-A-9 107 386 (SCHERING CORPORATION) * page 29, lines 1-8 * | 1-3 | A 61 K<br>C 07 K |

The present search report has been drawn up for all claims

| Place of search | Date of completion of the search | Examiner |
|---|---|---|
| BERLIN | 12-06-1992 | AVEDIKIAN P.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)